# EUROPEAN PATENT APPLICATION

(11) **EP 4 566 663 A1**
(43) Date of publication of application: **11.06.2025**
(21) Application number: 24216759.1
(22) Date of filing: 02.12.2024
(51) Int. Cl.: A61N 5/10

(54) **METHOD TO IMPROVE THE NON-COPLANAR PLANNING CAPACITY OF A COUCH THAT SUPPORTS ONLY LIMITED RANGE OF ROTATION**

(30) Priority: 05.12.2023 US 202318529041
(71) Applicant: Siemens Healthineers International AG, 6312 Steinhausen (CH)
(72) Inventor: KUUSELA, Esa, 02320 Espoo (FI)
(74) Representative: Mathisen & Macara LLP

(57) **Abstract**

Systems, devices, and methods for increasing the dose spread in treatment systems delivering non-coplanar treatments to a patient using a couch 112 with limited couch rotation angle ranges, the increasing including combining dose spreads generated by shifting ±L the isocenter I₀ location and shifting the couch angles ±α in such a way as to maximize the dose spread in both sides of the patient, and systems, devices and methods for generating non-coplanar treatment plans with different couch-kick ranges from which the non-coplanar treatment plan that takes into account the couch shifts needed for correcting patient positioning is selected for delivery.

## Description

### FIELD

The present disclosure relates generally to radiation therapy systems, devices, and planning methods, and more particularly to systems, devices, and planning methods for increasing the effective couch rotation angle range and dose spread in non-coplanar treatments where the possible couch rotation angle ranges are limited by the design of the couch model.

### BACKGROUND

Radiation therapy involves medical procedures that use external radiation beams to treat pathological anatomies (tumors, lesions, vascular malformations, nerve disorders, etc.) by delivering prescribed doses of radiation (X-rays, gamma rays, electrons, protons, and/or ions) to the pathological anatomy, while minimizing radiation exposure to the surrounding tissue and critical anatomical structures.

In general, a full radiotherapy planning and treatment workflow includes several phases: a treatment planning phase, a treatment delivery phase, and a monitoring and evaluating phase in which the progress of the treatment, e.g., the dose accumulation is monitored.

In the treatment planning phase, first a precise three-dimensional (3D) map of the anatomical structures in the area of interest (head, body, etc.) is constructed using any one of (or combinations thereof) a computed tomography (CT), cone-beam computed tomography (CBCT), magnetic resonance imaging (MRI), positron emission tomography (PET), 3D rotational angiography (3DRA), or ultrasound techniques. This determines the exact coordinates of the target within the anatomical structure, namely, locates the tumor or abnormality within the body and defines its exact shape and size. On these images, organs at risk (OARs) in the region of interest are also delineated. This is followed by a prescription step, where the level of radiation which should be delivered to the target (tumors) and the level of protection of the OARs which needs to be achieved to avoid side-effects for the patient is specified. In the prescription step, a motion path for the radiation beam is also computed to deliver a dose distribution to the target within a treatment volume that the radiation oncologist finds acceptable, considering a variety of medical constraints. Then, a team of specialists develop a treatment plan using special computer software to optimally irradiate the tumor and minimize dose to the surrounding normal tissue by designing beams of radiation to converge on the target area (target region) from different angles and planes.

In the treatment delivery phase, the radiation treatment plan is executed. During this phase, the radiation dose is delivered to the patient according to the prescribed treatment plan.

The primary objective in radiation therapy is to maximize dose to the target while minimizing dose to surrounding healthy tissue and organs at risk (OARs). As such, it is beneficial to spread the incoming dose to a larger volume of lower doses as much as possible. This is especially true for treatment plans where steep dose fall-off outside of the target is required, such as for stereotactic body radiotherapy (SBRT) and single-fraction radiosurgery (SRS). To spread the incoming dose, and thus provide a dose distribution that maximizes dose to the target and minimizes dose to surrounding healthy tissue and OARs, radiation therapies use coplanar and non-coplanar treatment techniques.

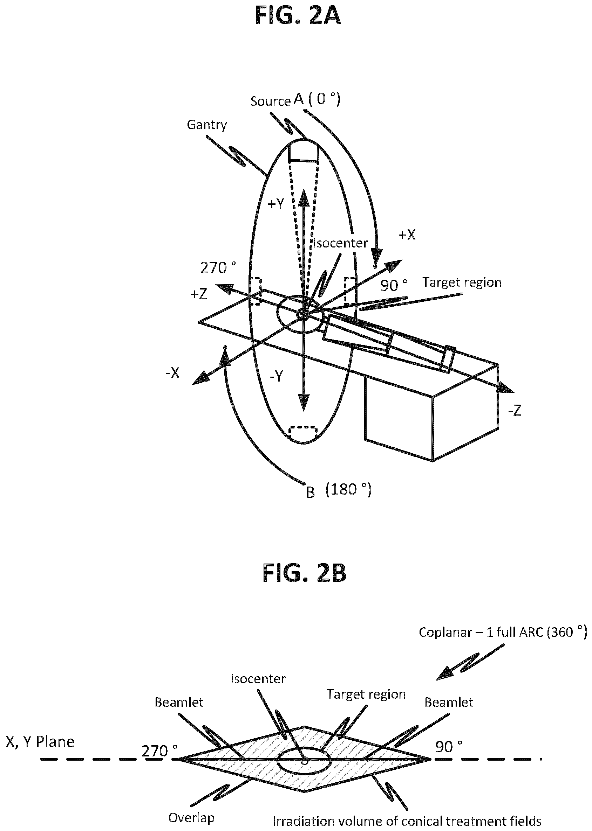

Coplanar treatment involves directing the radiation towards the target region from a plurality of different angles by rotating a source of radiation around the patient by use of a rotating gantry. An exemplary system delivering coplanar treatment is shown in FIG. 2A. The angles at which radiation is applied are selected such that each beam of radiation passes through the target region. In this way, a cumulative radiation dose may be built up in the target region over the course of a treatment arc in which the radiation source rotates through a certain angle. The radiation is emitted in a radiation plane which is co-incident with the plane of the gantry around which the radiation source rotates, and the radiation is delivered to a radiation isocenter at the center of the gantry regardless of the angle to which the radiation head is rotated around the gantry. In a coplanar field geometry, therefore, the dose is spread in the XY-plane of the patient coordinate system as shown in FIG. 2B.

In coplanar treatment, after the radiation source has been rotated 180 degrees, any subsequent radiation beams begin to pass through regions of healthy tissue, thus reducing the volume of healthy tissue available to spread the radiation dose. Restrictions are therefore generally imposed on radiation therapy systems delivering coplanar treatments.

Non-coplanar treatments are treatments that use various couch angles in combination with a range of gantry angles. This is generally done by rotating the couch on which the patient is positioned around the isocenter to a different position for each beam orientation. The couch angle is typically changed statically (i.e., while the beam is off) between the radiation fields.

Non-coplanar treatments generally use a number of fixed or rotating radiation beams that do not share the same geometric plane relative to the patient. This reduces the overlap in the regions away from the target, and therefore helps spread the unwanted dose spillage to a larger volume of lower dose while potentially escalating dose to the target. Using non-coplanar field geometry therefore improves the dose distribution of the treatment plan by increasing the spread of the incoming dose.

Since the non-coplanar treatment requires changing of the couch angle, the dose spread depends on the rotation angle range of the couch. Unfortunately, some couches support only a limited rotation angle range. For couches that were designed by the manufacturer to support a rotation angle range needed only to allow for adjusting the patient geometry after the patient has been positioned on the couch (i.e., using a small rotation of the couch to correct discrepancies between the patient coordinate system, as observed in the planning images, and the treatment machine coordinate system), the angle through which the couch can rotate is limited by design to about +/- 10 degrees. Such limited rotation angle range is not large enough to allow for a significant increase of the incoming dose spread. Such couches therefore do not support non-coplanar treatments.

Further, since couches with such limited rotation capabilities are generally used to correct patient positioning, in cases where the couch needs to be rotated to adjust for the positioning mismatch, the remaining available angle range through which the couch could be rotated is further restricted. Inversely, if a non-coplanar treatment plan is applied in a system using such couches, any planned non-zero couch angle reduces the freedom by which the patient positioning can be adjusted. Thus, for all practical purposes, such limited rotation angle couches do not support non-coplanar treatments, and therefore non-coplanar treatment plans are not developed for treatment systems that employ such treatment couches.

It would be therefore advantageous to be able to increase the effective rotation angle range of couches that have rotation angle ranges limited by couch designs so that non-coplanar treatments can be developed and applied in treatment systems that use such couches.

It would also be advantageous to increase the dose spread in non-coplanar treatments supported by such couches.

It would further be beneficial to generate non-coplanar treatments that would support the use of limited rotation angle range couches not only to increase the dose spread but also to adjust the patient positioning.

### SUMMARY

According to one aspect, the present invention provides a method according to claim 1. Optional features are specified in the dependent claims.

According to another aspect, the present invention provides apparatus according to claim 12.

According to a further aspect, the present invention provides a storage medium according to claim 13.

Systems and methods are disclosed for increasing dose spread in a radiation system delivering non-coplanar treatment to a target positioned on a couch having a limited couch rotation angle range.

In another aspect, the present invention provides a method of increasing dose spread in a radiation system delivering non-coplanar treatment to a target positioned on a couch having a limited couch rotation angle range, the method comprising:
generating a first dose spread by shifting an isocenter location of the radiation system;
generating a second dose spread by shifting the couch angle; and
combining the first dose spread generated by the shift in the isocenter location with the second dose spread generated by the shift in the couch angle.

In embodiments, the dose spread is increased by combining a first dose spread generated by shifting an isocenter location of the radiation system and a second dose spread generated by shifting the couch angle.

In embodiments, the combining is done in such a way as to maximize the dose spread in both sides of the target.

In embodiments, the shift in the isocenter location is obtained by moving the couch along a longitudinal axis, and the shift in the couch angle is obtained by rotating the couch about a vertical axis through the isocenter.

In embodiments, the first dose spread is generated by delivering a first radiation field at a first isocenter location and delivering a second radiation field at a second isocenter location, the first and second isocenter locations being shifted from the system isocenter location, and the second dose spread is generated by delivering the first and second radiation fields at different couch angles.

In embodiments, the combining includes splitting each of the first and second radiation fields at zero gantry angle of the radiation system into corresponding first and second arc segments, delivering the first and second arc segments of the first radiation field at the first isocenter location at different couch angles, and delivering the first and second arc segments of the second radiation field at the second isocenter location at different couch angles.

In embodiments, the shift between the different couch angles of the first and second arc segments is at the zero gantry angle of the radiation system.

In embodiments, the couch rotation angle range is limited by manufacturing design to ± 10 degrees.

Systems and methods are also disclosed for generating a plurality of non-coplanar treatment plans, each with a different couch-kick angle range, and for selecting a non-coplanar treatment plan from the plurality of non-coplanar treatment plans that accounts for couch angle shifts used for correcting target positioning errors.

A system for delivering a non-coplanar treatment to a patient positioned on a couch having limited rotation angle range is also disclosed.

In embodiments, the system comprises a gantry configured to rotate around the patient to deliver radiation to the target from different gantry angles, a couch configured to move in a longitudinal direction toward and away from the gantry and to rotate about a vertical axis through the system isocenter, and a processor optionally including a storage device configured to store a plurality of previously generated non-coplanar treatment plans, the processor being configured to increase a dose spread in the system by combining isocenter location shifts with couch angle shifts, e.g. by combining a first dose spread generated by shifting the isocenter location through couch movement with a second dose spread generated by shifting the couch angle.

In the system, the first dose spread may be generated by delivering a first radiation field at a first isocenter location and delivering a second radiation field at a second isocenter location, the first and second isocenter locations being shifted from the system isocenter location, and the second dose spread is generated by delivering the first and second radiation fields at different couch angles, the combining including: splitting each of the first and second radiation fields at zero gantry angle into corresponding first and second arc segments; delivering the first and second arc segments of the first radiation field at the first isocenter location at different couch angles; and delivering the first and second arc segments of the second radiation field at the second isocenter location at different couch angles, wherein the shifts between the different couch angles of the first and second arc segments is at the zero gantry angle of the radiation system.

In the system each of the first and second arc segments may be half arcs spanning between zero and 180 degrees around the gantry clockwise or counterclockwise.

In the system, the processor may be further configured to select a non-coplanar radiation treatment plan from the plurality of previously generated non-coplanar treatment plans based on an amount of angle shift needed for correcting the target positioning errors; and deliver the selected non-coplanar treatment plan.

A non-transitory computer-readable storage medium upon which is embodied a sequence of programmed instructions for increasing a dose spread in a system delivering non-coplanar treatment using a treatment couch with limited rotation angle range is also disclosed herein. Execution of the sequence of programmed instructions can cause the computer processing system to generate a first dose spread by delivering a first radiation field at a first isocenter location and delivering a second radiation field at a second isocenter location, the first and second isocenter locations being shifted from the system isocenter location, generate a second dose spread by delivering the first and second radiation fields at different couch angles, and combine the first and second dose spreads in such a way as to maximize the dose spread in both sides of the patient, the combining including: splitting each of the first and second radiation fields at zero gantry angle into corresponding first and second arc segments, delivering the first and second arc segments of the first radiation field at the first isocenter location at different couch angles, and delivering the first and second arc segments of the second radiation field at the second isocenter location at different couch angles. The shifts between the different couch angles of the first and second arc segments is at the zero gantry angle of the radiation system.

Objects and advantages of embodiments of the disclosed subject matter will become apparent from the following description when considered in conjunction with the accompanying drawings.

### BRIEF DESCRIPTION OF DRAWINGS

Embodiments will hereinafter be described with reference to the accompanying drawings, which have not necessarily been drawn to scale. Where applicable, some features may not be illustrated to assist in the illustration and description of underlying features.
FIG. 1 is a simplified schematic diagram of a radiation therapy system configured to deliver non-coplanar treatments using a treatment couch having a limited rotation range, according to various embodiments of the disclosed subject matter.
FIG. 2A is a schematic illustration of a radiation therapy system delivering coplanar radiation to a patient.
FIG. 2B is an illustration of beam arrangements in a coplanar field setting as imaged from above.
FIGS. 3A-3C are schematic illustrations of a radiation therapy system with a treatment couch configured to rotate around the isocenter through a limited rotation angle range, according to various embodiments of the disclosed subject matter.
FIG. 4 is a schematic illustration of a radiation therapy system delivering non-coplanar treatment by rotating the treatment couch around the isocenter, according to various embodiments of the disclosed subject matter.
FIG. 5 is an illustration of beam arrangements in a non-coplanar field setting as imaged from above.
FIG. 6A is a schematic illustration of a radiation therapy system with a treatment couch configured to move in a longitudinal direction, according to various embodiments of the disclosed subject matter.
FIGS. 6B-6E are schematic illustrations of isocenter location shifts obtained by the treatment couch movements in a longitudinal direction, according to various embodiments of the disclosed subject matter.
FIG. 7A is a schematic illustration of a radiation therapy system delivering radiation to shifted isocenter locations, according to various embodiments of the disclosed subject matter.
FIG. 7B is an illustration of beam arrangements in a shifted isocenter field setting as imaged from above.
FIG. 8A is a schematic illustration of a radiation therapy system configured to deliver non-coplanar treatment with increased dose spread, according to various embodiments of the disclosed subject matter.
FIG. 8B is an illustration of beam arrangements in a non-coplanar field setting with increased dose spread as imaged from above, according to various embodiments of the disclosed subject matter.
FIGS. 9A-9S and 9K'-9L' are schematic illustrations of gantry and treatment couch positions for increasing dose spread in non-coplanar treatments, according to various embodiments of the disclosed subject matter.
FIG. 10 is a schematic illustration of couch angle ranges and non-coplanar treatment plan variants created to support the usage of the limited couch rotation for both patent position correction and to non-coplanar delivery, according to various embodiments of the disclosed subject matter.
FIGS. 11-12 are schematic flow diagrams for increasing dose spread in non-coplanar treatments using treatment couches with limited rotation angle ranges, according to various embodiments of the disclosed subject matter.
FIG. 13 is a schematic flow diagram for generating and selecting treatment plan variants for non-coplanar treatments, according to various embodiments of the disclosed subject matter.

### DETAILED DESCRIPTION

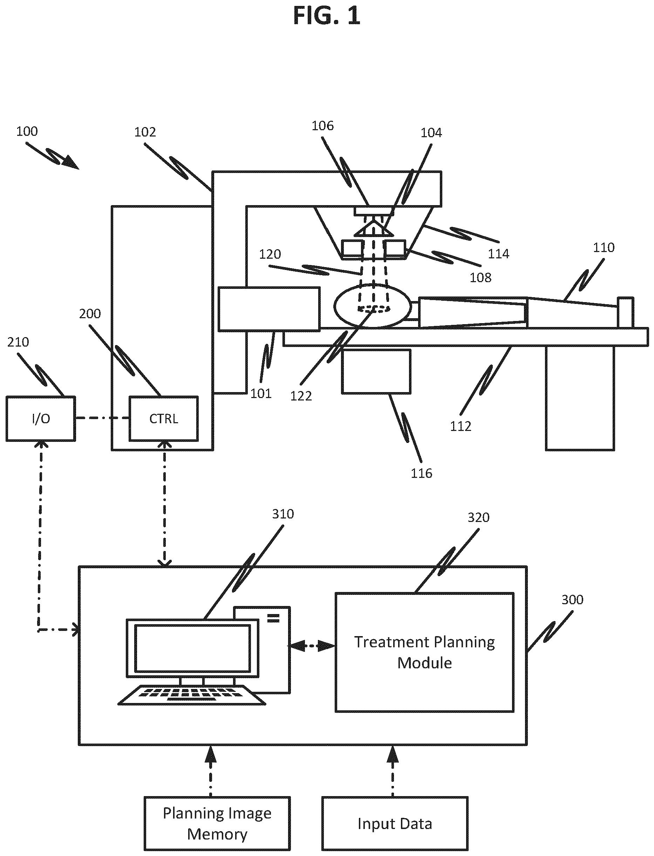

Referring to FIG. 1, an exemplary radiation therapy system 100 is shown, which can deliver non-coplanar treatments in accordance with the methods and techniques described herein. The radiation therapy system 100 can provide radiation to a patient 110 positioned on a patient support assembly generally known as a treatment couch 112 and can allow for the implementation of various non-coplanar radiation treatment protocols. The radiation therapy can include photon-based radiation therapy, particle therapy, electron beam therapy, or any other type of treatment therapy.

In an embodiment, the radiation therapy system 100 can include a radiation treatment device 101 such as, but not limited to, a LINAC operable to generate one or more beams of megavolt (MV) X-ray radiation for treatment. The LINAC may also be operable to generate one or more beams of kilovolt (kV) X-ray radiation, for example, for patient imaging. The system 100 has a gantry 102 supporting a radiation treatment head 114 with one or more radiation sources 106 and various beam modulation elements, such as, but not limited to, flattening filter 104 and collimating components 108. The collimating components 108 can include, for example, a multi-leaf collimator (MLC), upper and lower jaws, and/or other collimating elements. The collimating components 108 and/or the flattening filter 104 can be positioned within the radiation beam path by respective actuators (not shown), which can be controlled by controller 200.

The gantry 102 can be a ring gantry (i.e., it extends through a full 360° arc to create a complete ring or circle), but other types of mounting arrangements may also be employed. For example, a static beam, or a C-type, partial ring gantry, or robotic arm can be used. Any other framework capable of positioning the treatment head 114 at various rotational and/or axial positions relative to the patient 110 may also be used.

In an embodiment, the radiation therapy device is a MV energy intensity modulated radiation therapy (IMRT) device. The intensity profiles in such a system are tailored to the treatment requirements of the individual patient. The IMRT fields are delivered with MLC 108, which can be a computer-controlled mechanical beam shaping device attached to the head 114 and includes an assembly of metal fingers or leaves. For each beam direction, the optimized intensity profile is realized by sequential delivery of various subfields with optimized shapes and weights. From one subfield to the next, the leaves may move with the radiation beam on (i.e., dynamic multi-leaf collimation (DMLC)) or with the radiation beam off (i.e., segmented multi-leaf collimation (SMLC)). The MLC 108 therefore can be used to provide conformal treatment of tumors from various angles, as well as intensity modulated radiotherapy, whereby different radiation doses are delivered to different portions of the target region. The target region, namely, the irradiated volume proximate to the isocenter in the path of the X-ray beam, is defined by the jaws, the head 114, and the MLC 108. In IMRT, the leaves of the MLC are moved so that the treatment volume comprises the volume exposed during the course of the treatment.

Alternatively, or additionally, the radiation therapy device 101 can be a tomotherapy device, a helical tomotherapy device, or a simplified intensity modulated arc therapy (SIMAT) device, a volumetric modulated arc therapy (VMAT) device, or a volumetric high-definition (or hyperarc) therapy (HDRT). In effect, any type of radiotherapy device can be employed as the radiation therapy device 101 of system 100. Each type of radiation therapy device can be accompanied by a corresponding radiation plan and radiation delivery procedure.

The controller 200, which can be, but is not limited to, a graphics processing unit (GPU), can include a computer with appropriate hardware such as a processor, and an operating system for running various software programs and/or communication applications. The controller 200 can include a processing circuitry, a detector controller, a couch position controller, and a radiation source controller, each programmed and configured to achieve one or more of the functionalities described herein. The controller 200 may also include a microcontroller-based, single board computer, running a real-time operating system with acquisition, control, and interface software. It may also include high speed digital video interface card, a dedicated image processor card, and a parallel output to transmit the image data to an external image processor and display.

The controller 200 can include software programs that operate to communicate with the radiation therapy device 101, which software programs are operable to receive data from external software programs and hardware. The computer can also include any suitable input/output (I/O) devices 210, which can be adapted to allow communication between controller 200 and a user of the radiation therapy system 100, e.g., medical personnel. For example, the controller 200 can be provided with I/O interfaces, consoles, storage devices, memory, keyboard, mouse, monitor, printers, scanner, as well as a departmental information system (DIS) such as a communication and management interface (DICOM) for storing and transmitting medical imaging information and related data and enabling the integration of medical imaging devices such as scanners, servers, workstations, printers, network hardware, etc.

Alternatively, or additionally, the I/O devices 210 can provide access to a network (not shown) for transmitting data between controller 200 and remote systems. For example, the controller 200 can be networked via I/O 210 with other computers and radiation therapy systems. The radiation therapy system 100, the radiation treatment device 101, and the controller 200 can communicate with a network as well as databases and servers, for example, a treatment planning system 300. The controller 200 may also be configured to transfer medical image related data between different pieces of medical equipment.

The system 100 can also include a plurality of modules containing programmed instructions (e.g., as part of controller 200, or as separate modules within system 100, or integrated into other components of system 100), which instructions cause system 100 to perform different functions related to non-coplanar radiation therapy, as discussed herein, when executed. For example, the system 100 can include or communicate with a treatment planning module 320 of a treatment planning system 300 operable to generate non-coplanar treatment plans for the patient 110 based on a plurality of data input to the system by the medical personnel using computer 310, a patient positioning module operable to position and align the patient 110 with respect to a desired location, such as the isocenter of the gantry, for a particular radiation therapy treatment, a treatment couch positioning module operable to position the treatment couch at different locations and to rotate the treatment couch through a range of rotation angles, an image acquiring module operable to instruct the radiation therapy system and/or the imaging device to acquire images of the patient 110 prior to the radiation therapy treatment (i.e., pre-treatment/reference images used for treatment planning and patient positioning) and/or during the radiation therapy treatment (i.e., in-treatment session images), and to instruct the radiation therapy system 100 and/or the imaging device 101 or other imaging devices or systems to acquire images of the patient 110.

The modules can be written in the C or C++ programming language, for example. Computer program code for carrying out operations as described herein may be written in any programming language, for example, C or C++ programming language.

The treatment planning system 300 can be used to generate non-coplanar treatment plans for the radiation therapy system 100. The treatment planning system 300 includes program memories that contain processor executable instructions that, when executed by the processor 310, generate non-coplanar treatment plans that can be executed by a processing unit (e.g., controller 200) of the radiation therapy system 100. The treatment planning system 300 is configured to communicate with a planning image memory containing image data, and with a knowledge base which is a database or other information retrieval system that contains plan templates including clinical goals (CGs) and priorities for different anatomical structures, as well as knowledge-based information such as patient records (i.e., previous/existing treatment plans) that are similar to the current patient record, treatment types, etc.

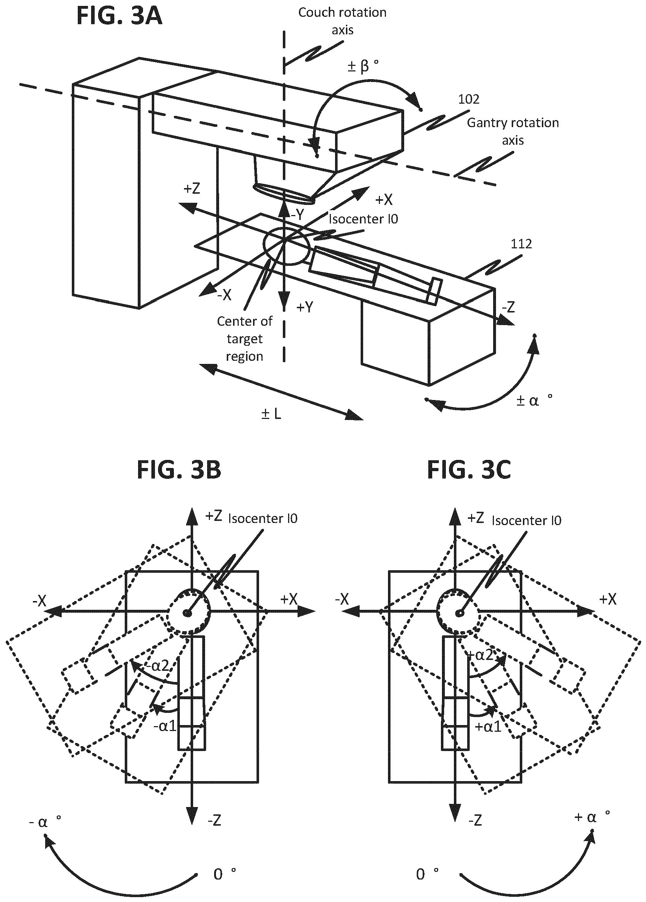

As shown in FIGS. 3A-3C, the treatment couch 112 can be positioned adjacent to the gantry 102 to place the patient 110 and the target region 122 within the range of operation for the radiation source 106. The gantry 102 can rotate clockwise or counterclockwise about a fixed rotation axis to place the radiation source 106 at any position within 360 degrees (β = ± 180°) around the target region 122. The full circle sweep (i.e., full 360 degree arc) lies in the radiation plane. The radiation plane is orthogonal to the fixed rotation axis and passes through the isocenter I₀. For isocentric treatment, the center of the target region 122 is aligned with the isocenter I₀ during a patient set up procedure.

The radiation therapy system 100 includes a rotation mechanism 116 coupled to a patient positioning system that allows for the treatment couch 112 to be rotated around a rotation axis that passes through the isocenter I₀. The system controller 200 can control the movement of the treatment couch 112 by communicating with the rotation mechanism 116 and the patient positioning system.

When the treatment couch 112 is in its original neutral position, the axis of rotation of the treatment couch 112 is substantially vertical (perpendicular to the plane of the floor and parallel with the vertical axis Y), and the treatment couch 112 can be rotated about the isocenter I₀ from an original zero angle position to rotated positions that are within ±α degrees of the zero angle position. The rotation angle range of the treatment couch 112 is limited to approximately ± 10 degrees around the neutral zero angle position as shown in FIGS. 3B-3C. This maximum rotation angle range is limited by the manufacturing design of the couch.

In its original neutral position, the longitudinal axis (i.e., Z axis) is parallel to the long side of the treatment couch 112, and the transverse axis (X axis) is parallel with the short end of the treatment couch 112. The treatment couch 112 is also configured to be moved along the longitudinal axis Z from an original neutral position to a distance that is ±L distance away from the original neutral position. As shown in FIG. 3A, when the treatment couch 112 is moved by a +L distance from its original neutral position, the treatment couch is moved toward the gantry 102 along the Z axis, and when the treatment couch 112 is moved by a -L distance from its original neutral position, the treatment couch 112 is moved away from the gantry 102 along the Z axis.

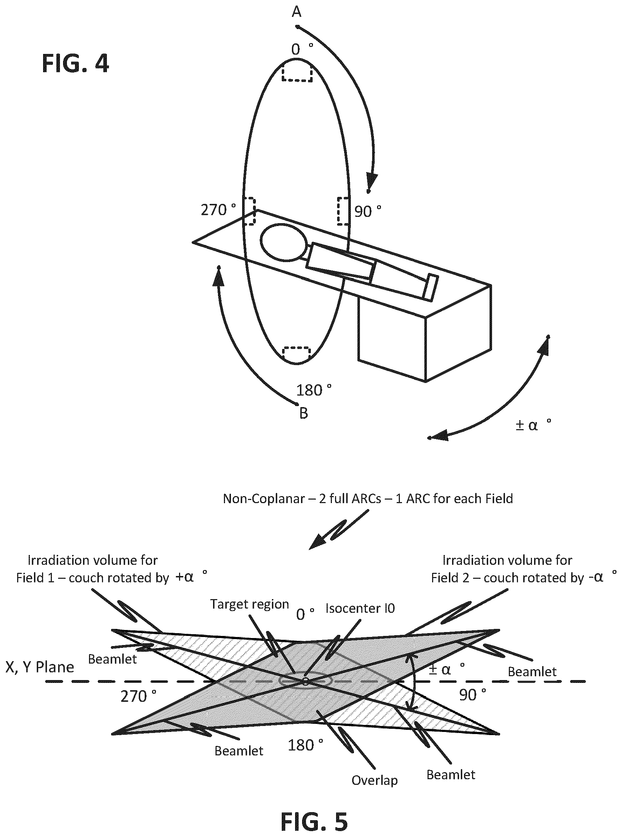

As shown in FIGS. 4 and 5, in a standard non-coplanar treatment plan (standard "couch-kick" plan), the treatment couch 112 is rotated around the isocenter I₀ in a first direction to a first rotated position +α₁°, and a full ARC (i.e., gantry rotated from zero to 360 degrees) of radiation 120 is delivered to the target 122 located at this first rotated position, then the treatment couch 122 is rotated around the isocenter I₀ in a second direction, that is opposite to the first direction, to a second rotated position -α₂°, and a full ARC (i.e., gantry rotated from zero to 360 degrees) of radiation is delivered to the target 122 located at this second rotated position. By simply rotating the target region 122 from a first rotational position to a second rotational position around the isocenter I₀, the beams of the two fields (i.e., the conical treatment fields obtained by the two full ARCs) are not entering the patient at the XY-plane. The overlap in the regions away from the target is therefore reduced. The reduced overlap helps spread the unwanted dose spillage to a larger volume of lower dose. As shown in FIG. 5, the amount of dose spread in the couch-kick plan depends on the gantry angle, reaching a nominal amount only when gantry is on the side (i.e., at 90 and 270 degrees), and disappearing entirely when the gantry is directly up or down (i.e., at 0 and 180 degrees).

In order for this couch-kick to be beneficial, there needs to be enough difference between the largest and smallest utilized couch angles. For a treatment couch that has a limited rotation angle range fixed by the couch design itself, the difference between the possible couch angles is not big enough for it to create a large enough dose spread for it to be beneficial. As such, for such couches, even if the largest possible rotation angles are utilized for +α₁ and -α₂, namely, ± 10 degrees, for example, the resulting dose spread is not large enough to be beneficial.

Reducing the overlap in the regions away from the target can also be done without rotating the treatment couch 112 by instead changing the isocenter location between the fields. Changing the isocenter location between the fields changes the parts of the conical fields that irradiate the target region 122. The farther the isocenter locations are from each other, the farther the conical fields are from each other, and a lesser portion of each field is used to irradiate the target region 122. This increase in the effective angular distance between the fields increases the dose spread and decreases the overlap.

Using different parts of the conical fields to increase the dose spread has numerous benefits. One such benefit is that a dose spread similar to a dose spread obtained with a +/- 8 degree couch angle shift can be obtained. Another benefit is that the obtained dose spread is even in all gantry angles, while in the traditional "couch-kick", the amount of dose-spread depends on the gantry angle. Also, the risk of collision between the treatment couch 112 and the gantry 102 and other parts of the radiation system 100 is smaller than in traditional couch-kick methods since the treatment couch 112 does not need to rotate.

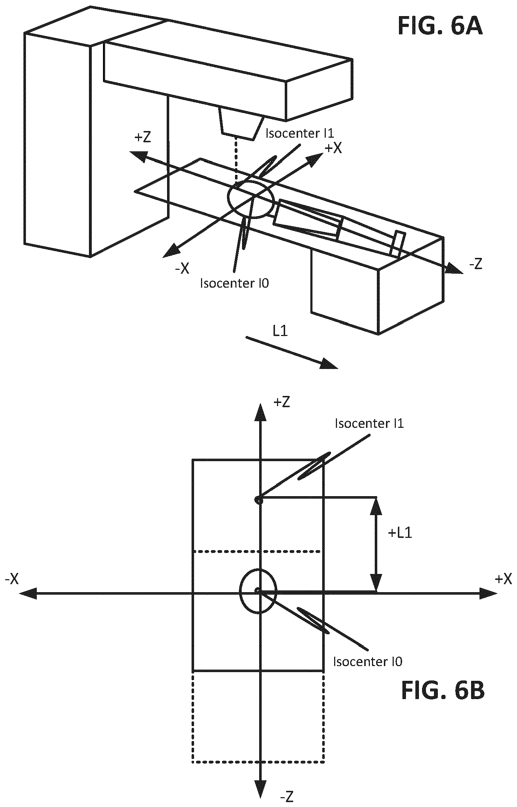

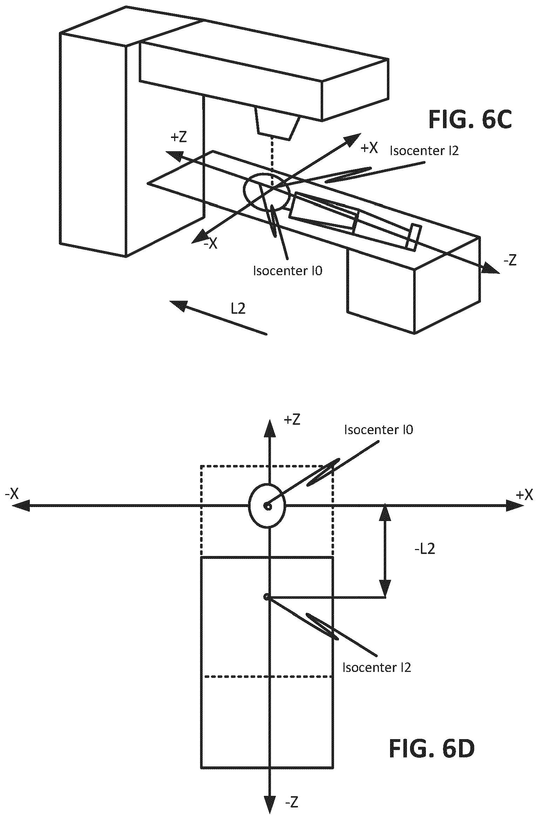

Moreover, for small target regions 122, the maximum field size is typically much larger than the target projection diameter. As such, it is possible to move the isocenter location to different positions using just the longitudinal axis of the couch, as shown in FIGS. 6A-6E.

As shown in FIGS. 6A and 6B, by moving the treatment couch 112 along the longitudinal axis -Z (away from the gantry 102) from its neutral original position by a distance L₁, for example, the center of the target region 122 moves from the gantry isocenter I₀ by a distance L₁, effectively shifting the isocenter location to a new isocenter location I₁. This is so because the moving of the treatment couch 112 moves the target region 122 from the first original position at the isocenter I₀ to a different position which is shifted relative to the original position.

By moving the treatment couch 112 along the longitudinal axis +Z (toward the gantry 102) from its neutral original position by a distance L₂, for example, the center of the target region 122 moves from the gantry isocenter I₀ by a distance L₂, effectively shifting the isocenter location to a new isocenter location I₂ as shown in FIGS. 6C-6D.

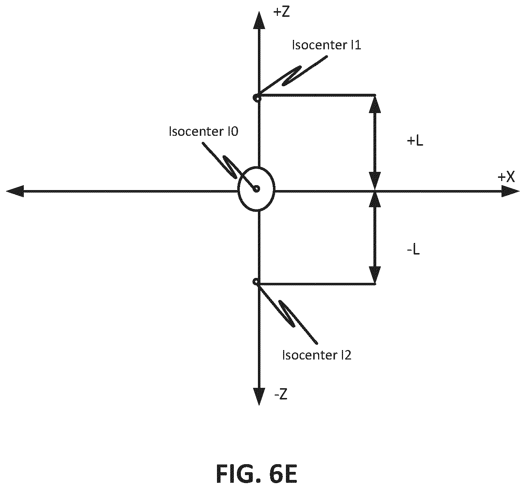

In an exemplary embodiment shown in FIG. 6E, the distance L1 is the same as the distance and L2 (except that the couch movement is in the opposite direction along the longitudinal axis). Thus, the amount of shift between the original isocenter location I₀ and the first isocenter location I₁ is the same as the amount of shift between the original isocenter location I₀ and the second isocenter location I₂.

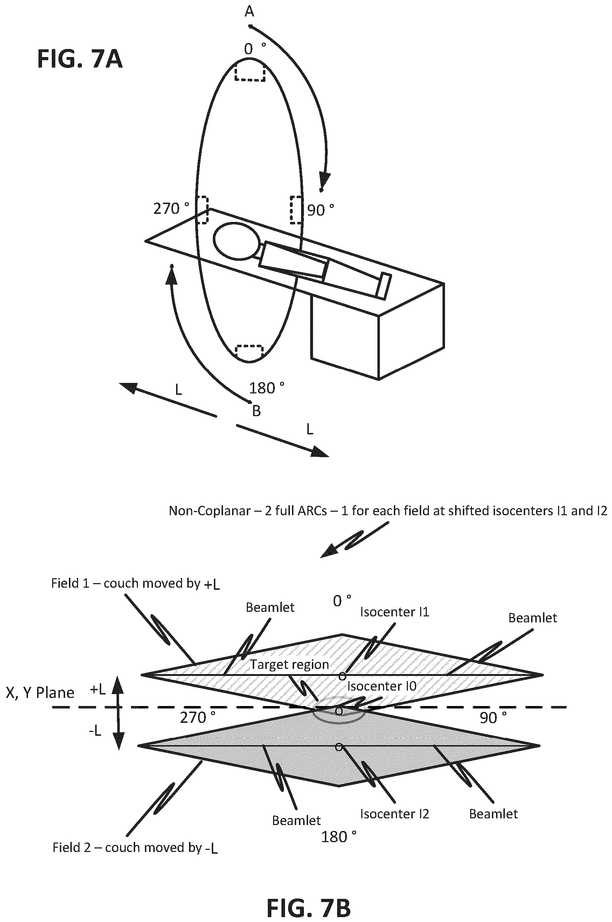

When a full ARC of radiation is applied to the first isocenter location I₁ by rotating the gantry 102 from its original 0 degree position to its final 360 degree position, and a second full ARC of radiation is applied to the second isocenter location I₂ by rotating the gantry 102 from its original 0 degree position to its final 360 degree position, as shown in FIG. 7A, a first and a second conical field is generated as shown in FIG. 7B. A portion of the first field (Field 1) covers a portion of the target region 122, and a portion of the second field (Field 2) covers another portion of the target region 122. Although the dose spread has increased, the increase is limited since the fields need to be close enough to adequately irradiate the target region 122.

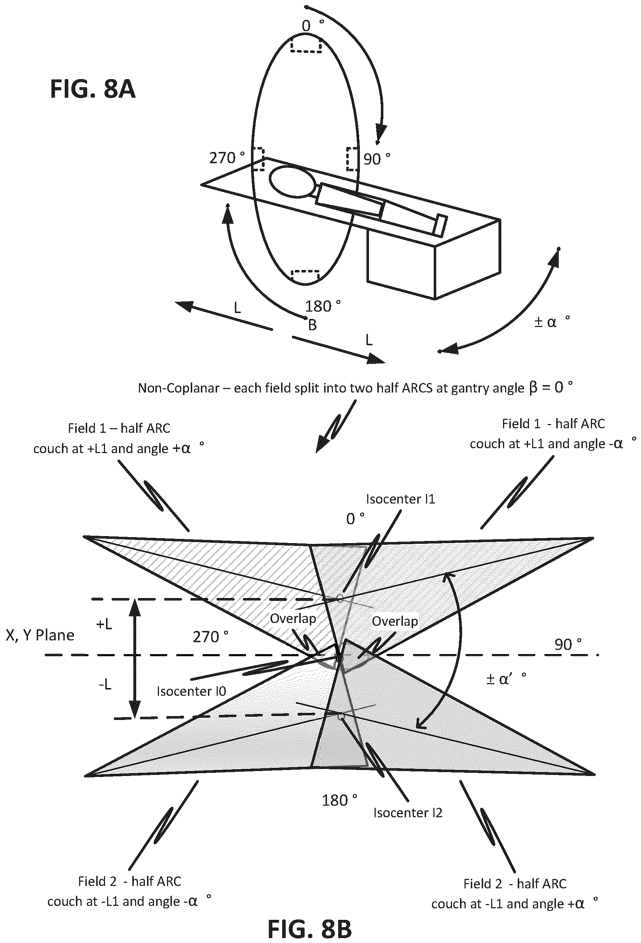

While the individual dose spread effects obtained using the couch-kick and isocenter shifting methods are relatively small, a method is described herein that combines these two effects, namely, combines the dose spread effect that can be obtained by rotating the treatment couch 112 and the dose spread effect that can be obtained by shifting the isocenter location through movement of the treatment couch 112 along the longitudinal axis, as shown in FIG. 8A.

A trivial combination of the two approaches would lead to a sub-optimal result, however, due to the different geometrical effects. For example, if the isocenter shifts and the couch angle shifts are combined in a way that the dose spread is maximized in the left side of the patient, the dose spreading effect will cancel out in the other side of the patient. Therefore, the combination of the isocenter shift and treatment couch rotation needs to be done in such a way as to maximize the dose spread in both sides of the patient.

The simplest solution is to split the original fields (Field 1 and Field 2) into two half-ARCs at gantry angle 0 (i.e., 0 to 180 degree gantry rotation clockwise for the first half-ARC, and 0 to 180 degree rotation counterclockwise for the second half-ARC), and use different couch angles for the right and left half-ARCs (i.e., +α° for the right half-ARC and -α° for the left half-ARC, for example), as shown in FIG. 8B. This way the spreading effect is maximized in both sides of the patient, namely, at 90 and 270 degree gantry angles, while at gantry angles 0 and 180, the patient benefits from the second effect, namely, the dose spread obtained by the shifting of the isocenter locations.

One exemplary way to combine the dose spreads for maximum dose spread at both side of the patient is to deliver the first half-ARC of the first radiation field (Field 1) at a first isocenter location I₁ (relative to the center of the target region 122) and a first treatment couch rotation angle +α°, deliver the second half-ARC of the first radiation field (Field 1) at the same first isocenter location I₁ but at a second couch rotation angle -α° that is opposite the first rotation angle +α°, and deliver the first half-ARC of the second radiation field (Field 2) at the isocenter location I₂ (relative to the center of the target region 122) and the first treatment couch rotation angle +α°, and deliver the second half-ARC of the second radiation field (Field 2) at the same second isocenter location I₂ but at the second couch rotation angle -α° which is opposite the first rotation angle +α°.

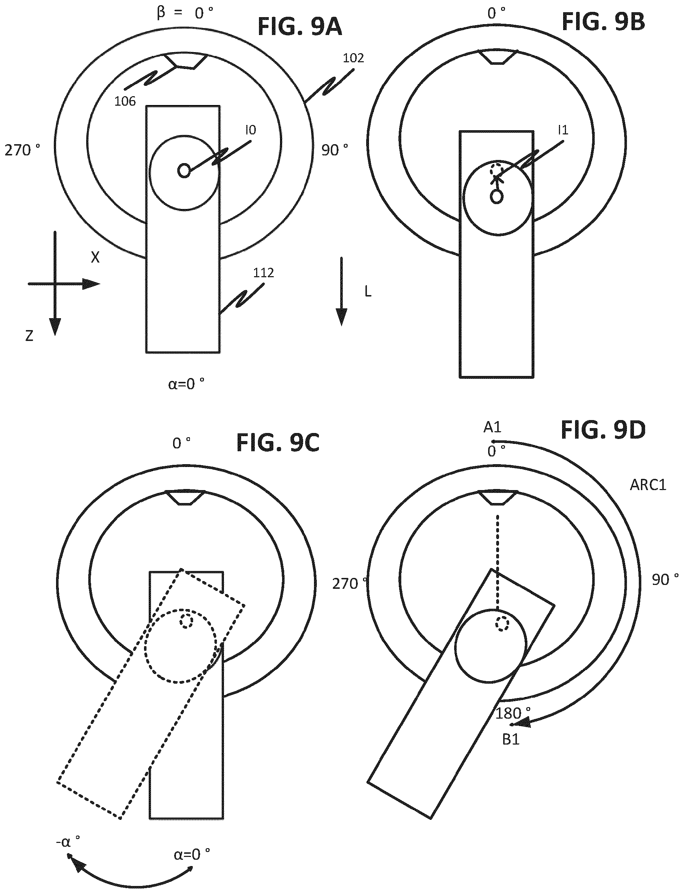

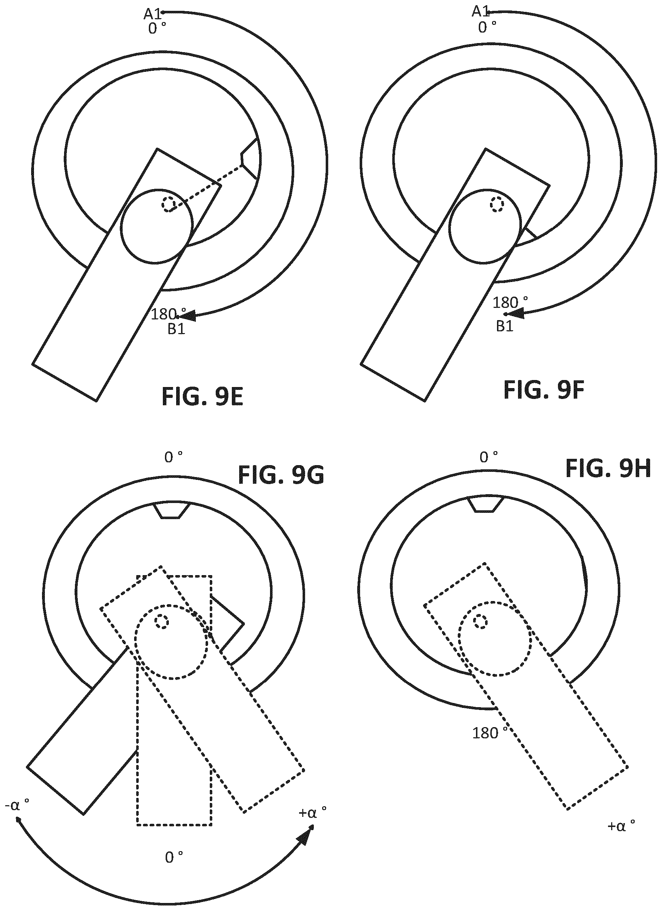

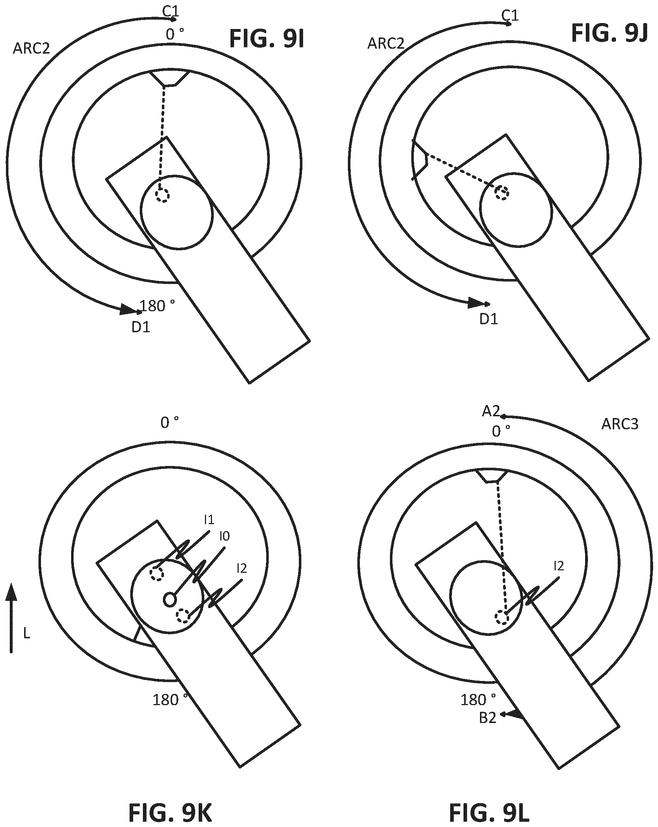

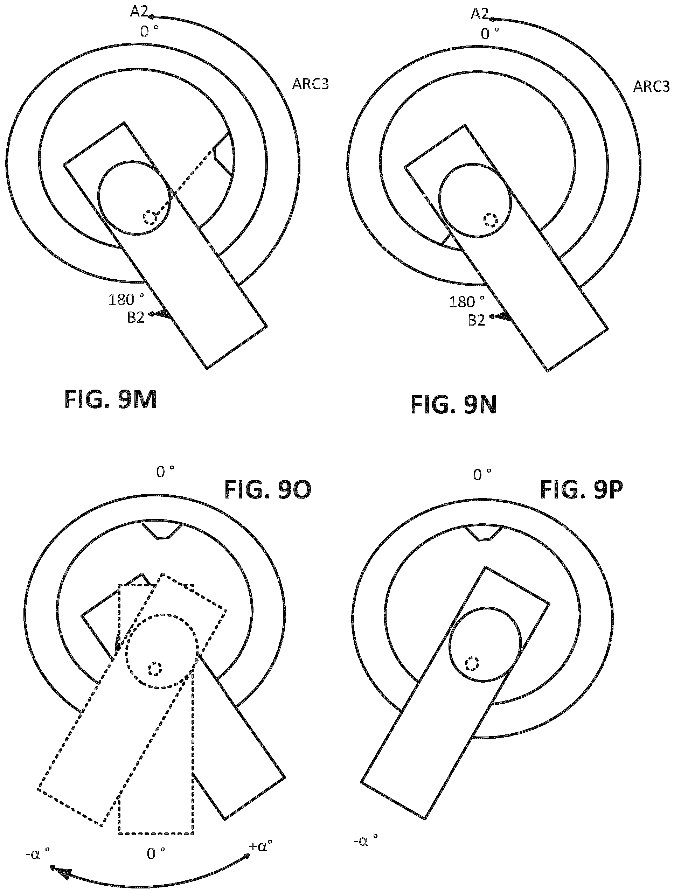

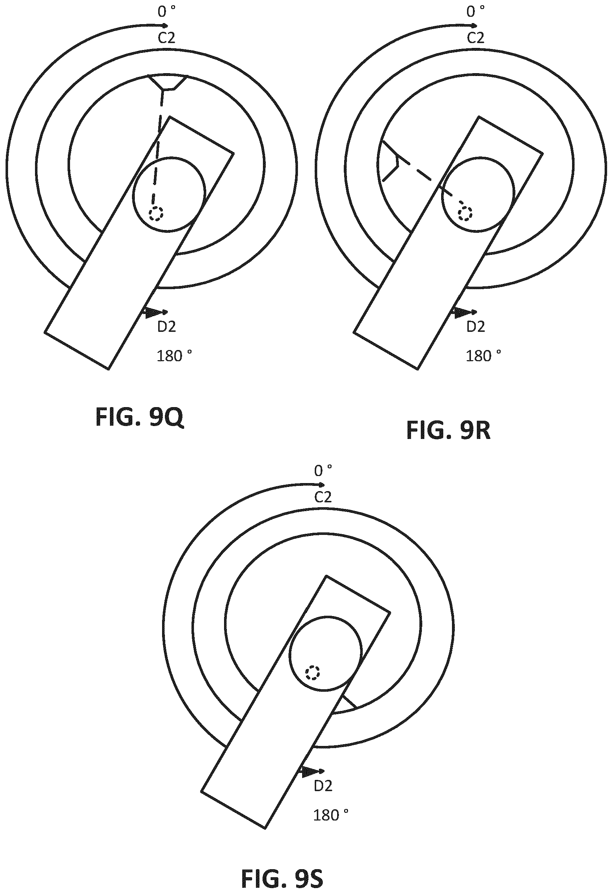

An exemplary way to combine the effects of the treatment couch 112 rotation and the isocenter shifts to obtain maximum dose spread at each side of the patient is shown in FIGS. 9A-9E. As shown in FIG. 9A, in neutral state, the gantry 102 is positioned at zero gantry angle (β = 0°), the treatment couch 112 is positioned at its neutral original position, and the center of the target region 122 is positioned at the gantry isocenter I₀. To obtain the first isocenter shift, the treatment couch 112 is moved along the longitudinal axis -Z to a first location that is moved from its original neutral location by a distance L. The move of the treatment couch 112 by a distance L from its neutral original position shifts the isocenter location from I₀ to a first isocenter location I₁, which is a distance L from the center of the target region 122, as shown in FIG. 9B.

The treatment couch 122 is next rotated to a first rotated position +α° as shown in FIG. 9C, and a first half-ARC (ARC1 from point A1 at 0°to point B1 at 180°) of a first radiation field (Field 1) is delivered onto the target region 122 by rotating the gantry 102 clockwise by 180 degrees (i.e., from 0 to 180 degrees) as shown in FIGS. 9D-9F. With the gantry angle back at 0°, the treatment couch 112 is next rotated to a second rotated position, which is at an angle +α° from its original zero angle neutral position, as shown in FIGS. 9G-9H. Then the second half-ARC (ARC2) of the first radiation field (Field 1) is delivered by rotating the gantry 102 in a counterclockwise direction from point C1 at 0°to point D1 at 180° as shown in in FIGS. 9I-9J.

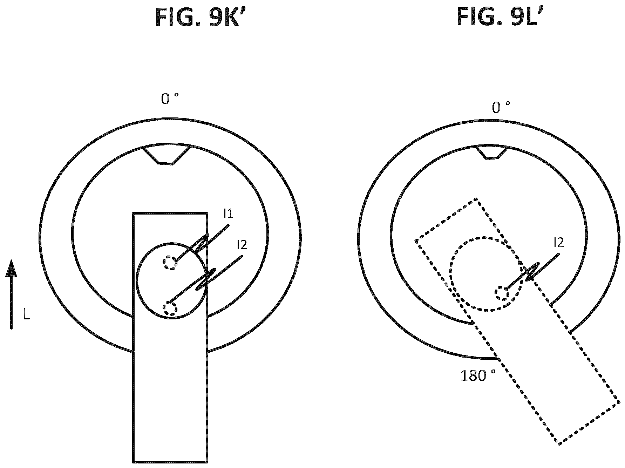

While the gantry is at 180°, the treatment couch 112 can be moved along the longitudinal axis +Z to a second location that is moved from its original neutral location by a distance L. The move of the treatment couch 112 by a distance L from its neutral original position shifts the isocenter location from I₀ to a second isocenter location I₂, which is a distance L from the center of the target region 122, as shown in FIG. 9K. The treatment couch 122 is still in its second rotated position, which is at an angle +α° from its original zero angle neutral position.

Then, a first half-ARC (ARC3 from point A2 at 0°to point B2 at 180°) of a second radiation field (Field 2) is delivered onto the target region 122 by rotating the gantry 102 clockwise by 180 degrees (i.e., from 0 to 180 degrees) as shown in FIGS. 9L-9N. With the gantry angle back at 0°, the treatment couch 112 is next rotated back to its first rotated position, which is at an angle -α° from its original zero angle neutral position, as shown in FIGS. 9O-9P. Then the second half-ARC (ARC3) of the second radiation field (Field 2) is delivered by rotating the gantry 102 in a counterclockwise direction from point C2 at 0°to point D2 at 180°) as shown in in FIGS. 9R-9S.

Although couch rotation angles ±α° have been indicated for the treatment couch rotation angles, the invention is not limited to these angles, and different couch rotation angles can be used for each of the first and second half-ARCs of both radiation fields (Field 1, Field 2) that are within the couch angle ranges permitted by the design of the treatment couch 112. To obtain maximum dose spread by the rotation of the treatment couch 112, however, the angles used for each of the first and second half-ARCs of each of the first and second radiation fields (Field 1, Field 2) may be the maximum rotation angles in each direction allowed by the couch design (i.e., ±10 degrees, for example).

Also, although the isocenter shift shown in FIG. 9K is at the 180° gantry angle while the treatment couch 112 is at the second rotated position +α°, this is an optional feature. Alternatively, the isocenter shift can be made after the gantry is moved to its 0° position and the treatment couch 112 has been shifted to its original zero position as shown in FIGS. 9K'-9L'.

In an exemplary embodiment, for an isocenter shift L of ± 12 cm and a couch rotation angle α° of ± 10°, an effective couch rotation angle α'° of ± 17° can be obtained as shown in FIG. 8B.

Although the exemplary combination method uses a full ARC for each field, that is exemplary only. Each field (i.e., Field 1, Field 2) can be less than a full ARC. Each of the corresponding ARC segments, therefore, do not need to be half-ARCs. Instead, each of the arc segments may encompass half of what the corresponding field ARC encompasses.

Furthermore, neither the order of the fields, nor the order of the ARC segments is limited to the exemplary combination method. Instead, the order of the fields and field segments may be arbitrary. For example, Field 2 may be delivered first and Field 1 second, and each of the corresponding first ARC segments may follow the corresponding second ARC segments. Any combination of radiation field delivery and couch treatment movement is contemplated as long as the couch rotation is at the 0° gantry angle, and the obtained dose spread is maximum at both sides of the patient. The method therefore can be generalized to arcs that are not full and the isocenter shifts are not exactly at the gantry angle 0°.

Other alternative combination methods include not splitting the radiation fields (Field 1, Field 2), but instead obtain a similar effect using a combination of dynamic couch angle shifts and/or dynamic isocenter shifts or utilizing more than two static couch angles to deliver fields around a single isocenter.

As discussed herein, a possible issue may arise if the non-coplanar treatment plan does not account for the amount of treatment couch rotation angle that is used up to reposition the patient (and thus the target region 122) prior to radiation delivery. Generally, during treatment planning, one or more planning images/image slices, such as CT, CBCT, MRI, etc. images/image slices, for example, of the portion of the patient that includes the tumor are obtained. Then a qualified medical personnel (physician) determines and delineates the target region on the image/image slices by generating a body contour around the total volume to be irradiated, as well as delineating the structures contained within the body contour, such as, one or more malignant tumors that are to receive therapeutic doses of radiation (i.e., target structures/volumes) and structures whose irradiation should be limited, since a dose of radiation in excess of a certain amount may adversely affect them (i.e., organs at risk (OARs)). The planning images used for the treatment planning can also be images that were previously taken and stored in a planning image memory.

Since the planning images are generated relative to a planning isocenter (i.e., isocenter of the imaging system that is used to generate the planning images), in order to properly deliver the radiation to the target region 122 during the treatment session using the radiation therapy system 100, the planning isocenter should be in the same location as the system isocenter I₀. If they are not, in order to adjust the isocenter positions, typically the treatment couch is moved in a transversal direction and is rotated to match the surrounding region as much as possible. The treatment couch 112 is moved and rotated until the misalignment between the planned and treatment system isocenter locations, and thus the misalignment between the planned patient geometry (i.e., patient coordinate system as observed in the planning image) and the actual patient geometry after positioning (i.e., patient coordinate system in the treatment coordinate system) is corrected.

Using a treatment couch 112 with limited rotation angle range to correct the patient geometry mismatch takes away some of the rotation angle range available for the couch-kick effect. This either further reduces the rotation angle range available for producing the couch-kick effect in non-coplanar treatments, or the non-coplanar treatment plans cannot be delivered.

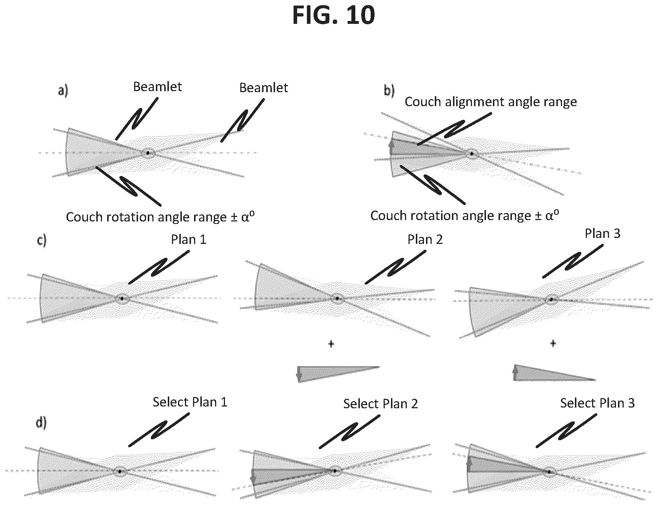

For example, as shown in FIGS. 10 (a) and (b), if the treatment couch 112 is limited to a +/- 10 degree couch rotation angle range, and the non-coplanar treatment plan utilizes +/- 7 degree couch-kicks (i.e., the treatment plan asks for the treatment couch angle to be shifted by +/- 7 degrees), it means that there is actually only +/- 3 degrees left to be used to adjust the patient position (i.e., the available couch alignment angle range). If the patient position mismatch correction requires a larger angle correction than the available +/- 3 degree couch alignment angle range, the non-coplanar treatment plan cannot be delivered.

With the increased effective couch rotation angle range (i.e., ±α' = ± 17 degrees) that can be obtained by combining the couch rotation angle shifts and isocenter location shifts, the risk that some non-coplanar treatment plans will not be able to be delivered is reduced.

An additional solution is to generate a plurality of alternative non-coplanar treatment plan variants for the patient 110 during the treatment planning phase instead of generating a single non-coplanar treatment plan for treatment delivery. Generally, non-coplanar treatment plans involve combining gantry rotation with treatment couch rotation so that beams of radiation converge on the target region from different angles and planes. This involves developing a set of radiation arcs with treatment couch rotation to obtain the desired radiation trajectories.

Each of the alternative non-coplanar treatment plan variants can be designed to utilize a different range of couch rotation angles (i.e., different couch kicks), as shown in FIG. 10C. For example, a first non-coplanar treatment plan can be generated that is designed to use a treatment couch rotation angle range of (-7, +7) degrees, a second non-coplanar treatment plan can be generated that is designed to use a treatment couch rotation angle range of (-13, +1) degrees, and a third non-coplanar treatment plan can be generated that is designed to use a treatment couch rotation angle range of (-1, +13) degrees.

After adjusting the patient positioning to correct for patient geometry mismatch, and prior to treatment delivery, the medical personnel can have the option to select between the plurality of non-coplanar treatment plans to be delivered, based on how much couch angle range is still available for couch-kick after the treatment couch 112 is adjusted to correct the patient positioning errors.

For example, if the correction is in a (-3, +3) degree angle range, the first non-coplanar treatment plan candidate is deliverable, since the remaining couch angle that could be used for the couch-kick falls within the (-10, +10) degree angle range that the treatment couch 112 has available. If the correction is in a (+3, +9) degree angle range, the second non-coplanar treatment plan is deliverable since a greater than 3 degrees correction added to the nominal couch angle of -13 degrees leads to an actual couch angle of -10 degrees or more, and any correction less than 9 degrees does not cause the couch angle to go out of the valid range. Similarly, if the correction is in a (-9, -3) degree range, the third treatment plan option would be deliverable.

It is to be understood that the specific numbers used for the couch angle ranges and corrections are exemplary only, and that the embodiments described herein are not limited to these values.

Software stored in the planning image memory and/or computer 310 of the treatment planning system 300 is configured to be loaded and processed in any conventional manner and is configured to be executed in order to generate the plurality of non-coplanar treatment plan variants.

The treatment planning software further includes software for translating the results of each of the non-coplanar treatment plan variants into instructions for operating the radiation therapy system 100 for controlling the elements of the radiation therapy system 100 that define the beam geometry and angle of irradiation (treatment couch, gantry, MLC, etc.) in order to deliver the selected non-coplanar radiation treatment plan to the patient 110.

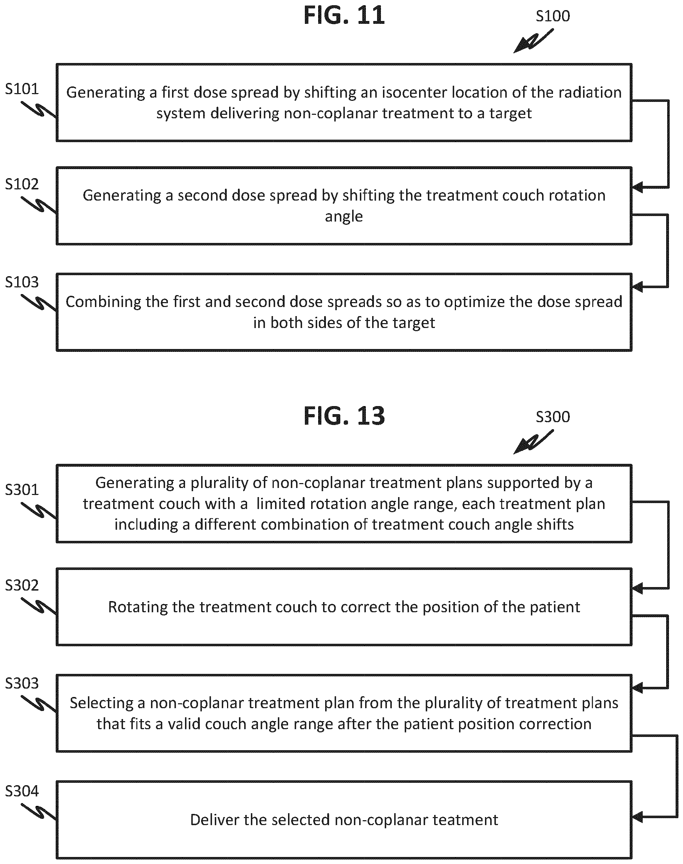

An exemplary process S100 for increasing the dose spread in a radiation system 100 delivering non-coplanar treatment to a patient 110 using a treatment couch 112 having a limited couch rotation angle range is shown in FIG. 11. In a first step S101, a first dose spread is generated by shifting an isocenter location of the radiation system delivering the non-coplanar treatment to the patient. In Step S102, a second dose spread is generated by shifting the treatment couch rotation angle, and in Step S103, the first dose spread generated in S101 is combined with the second dose spread generated in S102 in such a way as to maximize the dose spread in both sides of the patient.

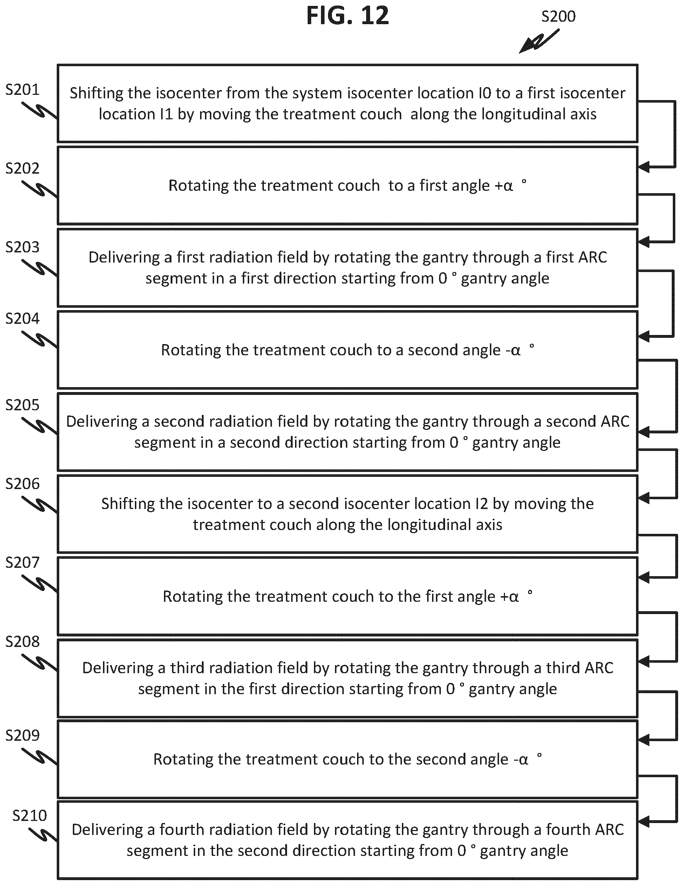

An exemplary process S200 of generating and combining the first and second dose spreads in order to maximize the dose spread in both sides of the patient 110 is shown in FIG. 12. In step S201, the isocenter is shifted from the system isocenter location I₀ to a first isocenter location I₁ by moving the treatment couch 112 along the longitudinal axis Z by a first distance L from a neutral original position. The neutral position is the position where the target region 122 in the patient is positioned at the system isocenter I₀. Then, the treatment couch 112 is rotated from its neutral position to a first angle position at +α degrees in S202. In S203, a first radiation field is delivered onto the target region 122 by rotating the gantry through a first ARC segment in a first direction starting from the zero gantry angle position. The first ARC segment may be a first half-ARC segment covered by the gantry angle moving from zero to 180 degrees in a clockwise direction. Then, with the gantry angle at zero, the treatment couch is rotated to a second rotated position at -α degrees in S204.

In S205, a second radiation field is delivered to the patient 110 by rotating the gantry through a second ARC segment in a second direction starting from the zero gantry angle. The second ARC segment may be a half-ARC segment covering 180 degrees in a counterclockwise direction. In S206, the isocenter location is shifted to a second isocenter location I₂ by moving the treatment couch 112 along the longitudinal axis by a distance L from its original neutral position, in a direction opposite to the first displacement. In S207, with the gantry angle at zero degrees, the treatment couch 112 is rotated to the first rotation position +α degrees. A third radiation field is then delivered to the patient 110 in S208 by rotating the gantry through a third ARC segment in the first direction starting from zero gantry angle. The third ARC segment may be a half-ARC segment covering 180 degrees in a clockwise direction. In S209, with the gantry angle at zero degrees, the treatment couch 112 is rotated to the second rotated position at -α degrees. A fourth radiation field is then delivered onto the patient 110 in S210 by rotating the gantry through a fourth ARC segment in the second direction starting from the zero gantry angle position. The fourth ARC segment may be a half-ARC covering 180 degrees in the counterclockwise direction.

FIG. 13 is an exemplary process of delivering non-coplanar treatment to patient 110 using a treatment couch with limited rotation angle range. In S301, a plurality of non-coplanar treatment plan variants are generated, each with a different combination of treatment couch angle shifts. In S302, and prior to commencing of the treatment delivery, the treatment couch 112 is rotated to correct the position of the patient in the radiation delivery system. Based on the amount of treatment couch rotation needed for patient positioning, a non-coplanar treatment plan is selected from the plurality of treatment plans in S303 that fits a valid couch angle range after the patient position correction. The selected non-coplanar treatment plan that is determined to be appropriate is then delivered to the patient in S304.

It is thus apparent that methods and systems are disclosed herein for increasing dose spread in a radiation system delivering non-coplanar treatment to a target positioned on a couch having a limited couch rotation angle range.

It is also apparent that methods and systems are disclosed herein for delivering a non-coplanar treatment to the target that also accounts for couch angle shifts used for correcting target positioning errors.

It is also apparent that systems including a computer processing device configured to execute a sequence of programmed instructions embodied on a computer-readable storage medium, the execution thereof causing the system to execute any or alternatively a combination of any of the method steps disclosed herein, are also disclosed.

A non-transitory computer-readable storage medium upon which is embodied a sequence of programmed instructions for increasing the dose spread and the effective couch rotation angle range in a system delivering non-coplanar treatment to a patient using a treatment couch with limited rotation angle range, and a computer processing system that executes the sequence of programmed instructions embodied on the computer-readable storage medium are also disclosed. Execution of the sequence of programmed instructions can cause the computer processing system to execute the dose spread and rotation angle increasing processes described herein.

A non-transitory computer-readable storage medium upon which is embodied a sequence of programmed instructions for of increasing dose spread in a radiation system delivering non-coplanar treatment to a target positioned on a couch having a limited couch rotation angle range, and a computer processing system that executes the sequence of programmed instructions embodied on the computer-readable storage medium are also disclosed.

It will be appreciated that the aspects of the disclosed subject matter can be implemented, fully or partially, in hardware, hardware programmed by software, software instruction stored on a computer readable medium (e.g., a non-transitory computer readable medium), or any combination of the above.

For example, components of the disclosed subject matter, including components such as a controller, process, or any other feature, can include, but are not limited to, a personal computer or workstation or other such computing system that includes a processor, microprocessor, microcontroller device, or is comprised of control logic including integrated circuits such as, for example, an application specific integrated circuit (ASIC).

Features discussed herein can be performed on a single or distributed processor (single and/or multi-core), by components distributed across multiple computers or systems, or by components co-located in a single processor or system. For example, aspects of the disclosed subject matter can be implemented via a programmed general purpose computer, an integrated circuit device, (e.g., ASIC), a digital signal processor (DSP), an electronic device programmed with microcode (e.g., a microprocessor or microcontroller), a hardwired electronic or logic circuit, a programmable logic circuit (e.g., programmable logic device (PLD), programmable logic array (PLA), field-programmable gate array (FPGA), programmable array logic (PAL)), software stored on a computer-readable medium or signal, an optical computing device, a networked system of electronic and/or optical devices, a special purpose computing device, a semiconductor chip, a software module or object stored on a computer-readable medium or signal.

When implemented in software, functions may be stored on or transmitted over as one or more instructions or code on a computer-readable medium. The steps of a method or algorithm disclosed herein may be embodied in a processor-executable software module, which may reside on a computer-readable medium. Instructions can be compiled from source code instructions provided in accordance with a programming language. The sequence of programmed instructions and data associated therewith can be stored in a computer-readable medium (e.g., a non-transitory computer readable medium), such as a computer memory or storage device, which can be any suitable memory apparatus, such as, but not limited to read-only memory (ROM), programmable read-only memory (PROM), electrically erasable programmable read-only memory (EEPROM), random-access memory (RAM), flash memory, disk drive, etc.

As used herein, computer-readable media includes both computer storage media and communication media, including any medium that facilitates transfer of a computer program from one place to another. Thus, a storage media may be any available media that may be accessed by a computer. By way of example, and not limitation, such computer-readable media may comprise RAM, ROM, EEPROM, CD-ROM or other optical disk storage, magnetic disk storage or other magnetic storage devices, or any other medium that may be used to carry or store desired program code in the form of instructions or data structures and that may be accessed by a computer.

Also, any connection is properly termed a computer-readable medium. For example, if the software is transmitted from a website, server, or other remote source using a transmission medium (e.g., coaxial cable, fiber optic cable, twisted pair, digital subscriber line (DSL), or wireless technologies such as infrared, radio, and microwave), then the transmission medium is included in the definition of computer-readable medium. Moreover, the operations of a method or algorithm may reside as one of (or any combination of) or a set of codes and/or instructions on a machine readable medium and/or computer-readable medium, which may be incorporated into a computer program product.

One of ordinary skill in the art will readily appreciate that the above description is not exhaustive, and that aspects of the disclosed subject matter may be implemented other than as specifically disclosed above. Indeed, embodiments of the disclosed subject matter can be implemented in hardware and/or software using any known or later developed systems, structures, devices, and/or software by those of ordinary skill in the applicable art from the functional description provided herein.

In this application, unless specifically stated otherwise, the use of the singular includes the plural, and the separate use of "or" and "and" includes the other, i.e., "and/or." Furthermore, use of the terms "including" or "having," as well as other forms such as "includes," "included," "has," or "had," are intended to have the same effect as "comprising" and thus should not be understood as limiting.

Any range described herein will be understood to include the endpoints and all values between the endpoints. Whenever "substantially," "approximately," "essentially," "near," or similar language is used in combination with a specific value, variations up to and including 10% of that value are intended, unless explicitly stated otherwise.

The terms "system," "device," and "module" have been used interchangeably herein, and the use of one term in the description of an embodiment does not preclude the application of the other terms to that embodiment or any other embodiment.

Many alternatives, modifications, and variations are enabled by the present disclosure. While specific examples have been shown and described in detail to illustrate the application of the principles of the present invention, it will be understood that the invention may be embodied otherwise without departing from such principles. For example, disclosed features may be combined, rearranged, omitted, etc. to produce additional embodiments, while certain disclosed features may sometimes be used to advantage without a corresponding use of other features. Accordingly, Applicant intends to embrace all such alternative, modifications, equivalents, and variations that are within the spirit and scope of the present invention.

## Claims

1. A method of treatment planning to increase dose spread in a radiation system delivering non-coplanar treatment to a target positioned on a couch having a limited couch rotation angle range, the method comprising providing a treatment plan to:
generate a first dose spread by shifting an isocenter location of the radiation system;
generate a second dose spread by shifting the couch angle; and
combine the first dose spread generated by the shift in the isocenter location with the second dose spread generated by the shift in the couch angle.

2. The method of claim 1, wherein the combining is such that the dose spread is maximized in both sides of the target.

3. The method of claim 2, wherein the maximized dose spread includes a dose spread that is maximum at 90 and 270 degree gantry angles of the radiation system.

4. The method of claim 1, 2 or 3, wherein the shift in the isocenter location is obtained by moving the couch along a longitudinal axis, and the shift in the couch angle is obtained by rotating the couch about a vertical axis through the isocenter.

5. The method of claim 1, 2, 3 or 4, wherein the first dose spread is generated by delivering a first radiation field at a first isocenter location and delivering a second radiation field at a second isocenter location, the first and second isocenter locations being shifted from the system isocenter location.

6. The method of claim 5, wherein the second dose spread is generated by delivering the first and second radiation fields at different couch angles.

7. The method of claim 6, wherein the combining includes:
splitting each of the first and second radiation fields at zero gantry angle of the radiation system into corresponding first and second arc segments;
delivering the first and second arc segments of the first radiation field at the first isocenter location at different couch angles; and
delivering the first and second arc segments of the second radiation field at the second isocenter location at different couch angles.

8. The method of claim 7, wherein the shifts between the different couch angles of the first and second arc segments is at the zero gantry angle of the radiation system.

9. The method of claim 8, wherein each of the first and second arc segments are half arcs spanning between zero and 180 degrees around the gantry clockwise or counterclockwise, and, optionally:
wherein the first arc segment of the first radiation field is a first half arc spanning between zero and 180 degrees clockwise, the second arc segment of the first radiation field is a second half arc spanning between zero and 180 degrees counterclockwise, the first arc segment of the second radiation field is a first half arc spanning between zero and 180 degrees clockwise or between 180 degrees and zero degrees clockwise, and the second arc segment of the second radiation field is a second half arc spanning between zero and 180 degrees counterclockwise or between zero and 180 degrees clockwise.

10. The method of claim 8 or 9, wherein the couch rotation angle range is limited by manufacturing design to ± 10 degrees, and, optionally:
wherein the combined dose spread increases an effective couch rotation angle range to ± 17 degrees.

11. The method of claim 8, 9 or 10, wherein the delivering of the non-coplanar treatment to the target accounts for couch angle shifts used for correcting target positioning errors, and, optionally:
wherein the delivering of the non-coplanar treatment includes delivering a non-coplanar treatment plan that takes into account the couch angle shifts used for correcting the target positioning errors, the method, optionally further comprising selecting the non-coplanar treatment plan from a plurality of previously generated treatment plans, the selection being based on the amount of angle shift needed for correcting the target positioning errors.

12. A system for delivering a non-coplanar treatment to a patient positioned on a couch having limited rotation angle range to increase dose spread in a, the system being configured to:
generate a first dose spread by shifting an isocenter location of the radiation system;
generate a second dose spread by shifting the couch angle; and
combine the first dose spread generated by the shift in the isocenter location with the second dose spread generated by the shift in the couch angle; and,
optionally, wherein the system is configured to perform the method of any one of claims 1 to 11.

13. A non-transitory computer-readable storage medium upon which is embodied a sequence of programmed instructions for increasing a dose spread in a system delivering non-coplanar treatment using a treatment couch with limited rotation angle range, which when executed by a processor cause the processor to:
generate a first dose spread by shifting an isocenter location of the radiation system;
generate a second dose spread by shifting the couch angle; and
combine the first dose spread generated by the shift in the isocenter location with the second dose spread generated by the shift in the couch angle; and,
optionally, wherein the instructions cause the processor to perform the method of any one of claims 1 to 11.

14. A system for delivering a non-coplanar treatment to a patient positioned on a couch having limited rotation angle range, the system comprising:
a gantry configured to rotate around the patient to deliver radiation to the target from different gantry angles;
the couch being configured to move in a longitudinal direction toward and away from the gantry, and to rotate about a vertical axis through the system isocenter; and
a processor including:
a storage device configured to store a plurality of previously generated non-coplanar treatment plans; and
a controller configured to control movement and rotation of the couch and rotation of the gantry around the patient;
the processor being configured to increase a dose spread in the system by combining a first dose spread generated by shifting the isocenter location through couch movement with a second dose spread generated by shifting the couch angle.

15. A non-transitory computer-readable storage medium upon which is embodied a sequence of programmed instructions for increasing a dose spread in a system delivering non-coplanar treatment using a treatment couch with limited rotation angle range, which when executed by a processor cause the processor to:
generate a first dose spread by delivering a first radiation field at a first isocenter location and delivering a second radiation field at a second isocenter location, the first and second isocenter locations being shifted from the system isocenter location,
generate a second dose spread by delivering the first and second radiation fields at different couch angles, and
combine the first and second dose spreads in such a way as to maximize the dose spread in both sides of the patient,
the combining including:
splitting each of the first and second radiation fields at zero gantry angle into corresponding first and second arc segments,
delivering the first and second arc segments of the first radiation field at the first isocenter location at different couch angles, and
delivering the first and second arc segments of the second radiation field at the second isocenter location at different couch angles,
wherein the shifts between the different couch angles of the first and second arc segments is at the zero gantry angle of the radiation system.
